# EUROPEAN PATENT APPLICATION

(11) **EP 4 338 691 A1**
(43) Date of publication of application: **20.03.2024**
(21) Application number: 21940633.7
(22) Date of filing: 31.12.2021
(51) Int. Cl.: A61B 17/22

(54) **THROMBECTOMY DEVICE**

(30) Priority: 17.05.2021 CN 202110532990; 17.05.2021 CN 202110532862
(71) Applicant: GUANGZHOU BOSSH MEDCIAL TECHNOLOGY CO. LTD., Guangzhou, Guangdong 510555 (CN)
(72) Inventor: SHEN, Bin, Shanghai 200124 (CN)
(74) Representative: Wang, Bo
(86) International application number: PCT/CN2021/143852
(87) International publication number: WO 2022/242191

(57) **Abstract**

This application provides a thrombectomy device, including a cutter net and a cutter head. The cutter net is fixed to a front end surface of a catheter. The cutter head is located inside the catheter. A hollowed-out portion is arranged on the cutter net in an axially-penetrating manner. The cutter head is drivable to rotate in the catheter. A front end of the cutter head cooperates with the cutter net to form a cutting structure to excise a thrombus sucked from the hollowed-out portion. The cutting structure includes a first cutter edge and a second cutter edge. The first cutter edge is located on the cutter head, and the second cutter edge is located on the cutter net. When the thrombectomy device is operated, the first cutter edge rotates, the second cutter edge is fixed, and the first cutter edge and the second cutter edge cooperate to excise the thrombus. The thrombectomy device provided by this application has the advantages of good thrombectomy effect and the like.

## Description

### TECHNICAL FIELD

This application relates to the field of medical devices, and more particularly to a thrombectomy device.

### BACKGROUND

A thrombus is a clot of blood flow formed on the surface of an internal exfoliation or repair of a blood vessel in a cardiovascular system, and is composed of insoluble fibrin, deposited platelets, accumulated white blood cells, and trapped red blood cells. The thrombus causes local cerebral blood flow decrease or blood supply interruption, and cerebral ischemia and hypoxia lead to softening and necrosis, which causes focal nervous system symptoms, thus resulting in atherosclerotic cerebral or myocardial infarction. When the thrombus occurs, the activities of nerve center and important physiological functions are threatened due to local tissue ischemia due to vascular occlusion. If the occlusion in the blood vessel cannot be removed in time to make the blood flow unobstructed, the brain tissue in the ischemic area will suffer from dysfunction or loss of function, and even seriously threaten the life of patients. However, medical treatment is widely used in clinic at present, which cannot quickly remove the thrombus and the obstruction. The dilatation, anticoagulation, and thrombolytic therapy not only have poor curative effect, but also have the limitation of treatment time window, and even easily lead to serious consequences of tissue bleeding. The treatment of thrombosis is still a difficult problem in clinical medicine.

### SUMMARY

Based on the above situation, a main object of this application is to provide a thrombectomy device.

In order to achieve the above object, this application adopts the following technical solution. A thrombectomy device for excising a thrombus in a blood vessel includes a catheter and an excision assembly. The excision assembly includes a cutter net and a cutter head. The cutter net is fixed to a front end surface of the catheter, and the cutter head is located inside the catheter. A hollowed-out portion is arranged on the cutter net in an axially-penetrating manner. The cutter head is drivable to rotate in the catheter. A front end of the cutter head cooperates with the cutter net to form a cutting structure to excise a thrombus sucked from the hollowed-out portion. The cutting structure includes a first cutter edge and a second cutter edge. The first cutter edge is located on the cutter head, and the second cutter edge is located on the cutter net. The cutter head includes at least one cutter edge body. A front end of the cutter edge body closest to the cutter net is a first surface, and an edge of the first surface forms the first cutter edge. A partial edge of the hollowed-out portion close to the cutter head forms the second cutter edge. When the thrombectomy device is operated, the first cutter edge rotates, the second cutter edge is fixed, and the first cutter edge and the second cutter edge cooperate to excise the thrombus.

Preferably, the cutter edge body includes a second surface adj acent to the first surface. The second surface is arranged on the rear side of the first surface in a rotation direction of the cutter head, and the second surface is inclined away from the cutter net in a direction opposite to the rotation direction of the cutter head.

Preferably, the cutter head includes at least two cutter edge bodies. The at least two cutter edge bodies are distributed centrosymmetrically about a rotation axis of the cutter head. A space for removing the excised thrombus is provided between the adjacent two cutter edge bodies in the rotation direction of the cutter head.

Preferably, the cutter edge body and the space for removing the excised thrombus extend spirally in an axial direction of the catheter.

Preferably, the cutter head is a semicircular ring-like body. The first surface has a first edge, a second edge, a third edge, and a fourth edge, which are connected in sequence. The second edge and the fourth edge are arc-shaped, and the first edge and the third edge are connected to both ends of the second edge and the fourth edge respectively. The first edge and the third edge form the first cutter edge.

Preferably, the cutter head has a first plane and a second plane extending in an axial direction of the cutter head, and a cambered surface connecting the first plane and the second plane. The ends of the first plane and the second plane close to the cutter net are provided with a notch separately. A tip structure is formed on the side of the notch close to the cutter net. The tip structure includes a tip that forms the first cutter edge.

Preferably, the first surface is parallel to a reference plane A or an included angle is formed between the first surface and the reference plane A. The reference plane A is perpendicular to a rotation axis of the cutter head. The first surface is a plane. The first cutter edge is a part of the cutter head closest to the cutter net.

Preferably, the cutter net includes a cutter net body and a fixing member. The cutter net body is fixedly connected to the fixing member, and the cutter net is fixed to the catheter through the fixing member. The cutter net body includes an outer annular structure and at least two connecting ribs. The connecting ribs extend in a radial direction of the catheter, and radial outer ends of the connecting ribs are connected to the outer annular structure. The hollowed-out portion is formed between the adjacent connecting ribs. The connecting ribs and the cutter head cooperate to form the cutting structure. Partial edges of the connecting ribs close to the cutter head form the second cutter edge.

Preferably, the cutter net includes at least two fixing members. One end of each fixing member is fixedly connected to the outer annular structure, the other end of the fixing member is a free end, the distance from the fixing member to the axis of the cutter net is gradually reduced from the connecting end of the fixing member with the cutter net body to the free end. Clamping grooves quantitatively matched with the fixing members are provided on an outer wall of the catheter, and the depth of the clamping grooves is gradually increased from the end close to the cutter net to the end far away from the cutter net in the axial direction of the catheter. When the fixing members are fixed in the clamping grooves, the fixing members are clamped on the catheter.

Preferably, the cutter head and the cutter net are provided with a central through hole in the axial direction of the catheter for allowing passage of a guide wire.

Preferably, the thrombectomy device further includes a suction assembly. The suction assembly is communicated with an inner cavity of the catheter and sucks air from the catheter to make the interior of the catheter in a negative pressure state when the thrombectomy device is operated. The excision assembly further includes a power source that drives the cutter head to rotate in the catheter.

Preferably, the excision assembly includes a transmission shaft. The power source and the cutter head are connected through the transmission shaft. Before the thrombus is excised, a certain distance is provided between the cutter head and the cutter net, and the distance is less than or equal to 1 mm. When the thrombus is excised, the cutter head approaches the cutter net and forms the cutting structure for excising the thrombus.

Preferably, the speed of rotation of the cutter head is 60,000 rpm to 200,000 rpm when the cutter head excises the thrombus.

In the thrombectomy device provided by this application, the cutter net and the cutter head have simple structural design and cooperate well, thus improving the excision effect of thrombectomy. Specifically, the cutter net and the cutter head cooperate to form the cutting structure for excising the thrombus. The first cutter edge of the cutting structure is located on the cutter head, and the second cutter edge of the cutting structure is located on the cutter net. The cutter head includes at least one cutter edge body. The surface of the cutter edge body closest to the cutter net is the first surface, and the edge of the first surface forms the first cutter edge. The thrombus can be effectively excised by the cutting structure. Since the cutter net is fixed to the front end surface of the catheter, the thrombectomy device provided by this application can still effectively excise the thrombus without being affected by the thrombus size under the condition that the thrombus size is large and the blood vessel is about to be blocked or has been blocked. In the cutting structure, the first cutter edge rotates and the second cutter edge is fixed to form a dynamic shear structure, which can obtain the effect of efficiently excising the thrombus.

Other beneficial effects of this application will be described in the Detailed Description through the introduction of specific technical features and technical solutions. Those skilled in the art should be able to understand the beneficial technical effects brought by the technical features and the technical solutions through the introduction thereof.

### BRIEF DESCRIPTION OF THE DRAWINGS

Preferred implementations of a thrombectomy device according to this application will be described below with reference to the accompanying drawings. In the drawings:
FIG. 1 is a three-dimensional view of a thrombectomy device according to this application.
FIG. 2 is a schematic exploded view of a thrombectomy device according to this application.
FIG. 3 is a three-dimensional view of a first catheter in a thrombectomy device according to this application.
FIG. 4 is a front view of a first catheter in a thrombectomy device according to this application.
FIG. 5 is a cross-sectional view of a first catheter in a thrombectomy device according to this application.
FIG. 6 is an exploded view of an excision assembly in a thrombectomy device according to this application.
FIG. 7 to FIG. 9 are schematic structural diagrams of a transmission shaft in a thrombectomy device according to this application.
FIG. 10 is a schematic diagram of a three-dimensional structure of a cutter head in a thrombectomy device according to this application.
FIG. 11 is a front view of a cutter head in a thrombectomy device according to this application.
FIG. 12 is a side view of a cutter head in a thrombectomy device according to this application.
FIG. 13A and FIG. 13B are schematic diagrams of a three-dimensional structure of another cutter head in a thrombectomy device according to this application under different perspectives.
FIG. 14A and FIG. 14B are side views of the cutter head of FIG. 13A at different angles.
FIG. 15 is a three-dimensional view of a cutter net in a thrombectomy device according to this application.
FIG. 16 is a rear view of a cutter net in a thrombectomy device according to this application.
FIG. 17 is a schematic diagram of a deformed structure of a cutter net in a thrombectomy device according to this application.
FIG. 18 is a schematic diagram of a deformed structure of a cutter net body in a thrombectomy device according to this application.
FIG. 19 and FIG. 20 are schematic structural diagrams of thrombectomy of a thrombectomy device according to this application.

### DETAILED DESCRIPTION

The present utility model is described below based on embodiments but the present utility model is not limited to the embodiments. In the following detailed description of the present utility model, some specific details are described in detail. In order to avoid confusing the essence of the present utility model, the well-known methods, processes, flows, and components are not described in detail.

Furthermore, it will be understood by those of ordinary skill in the art that the drawings provided herein are for purposes of illustration and are not necessarily drawn to scale.

Unless the context expressly requires, the terms "including", "comprising", and the like throughout the specification and the claims shall be construed in the meaning of inclusion and not in an exclusive or exhaustive sense. That is to say, it means "including but not limited to".

In the description of the present utility model, it should be understood that the terms "first", "second", and the like are used for descriptive purposes only and cannot be understood to indicate or imply relative importance. Furthermore, in the description of the present utility model, unless stated otherwise, the meaning of "a plurality of" is two or more than two.

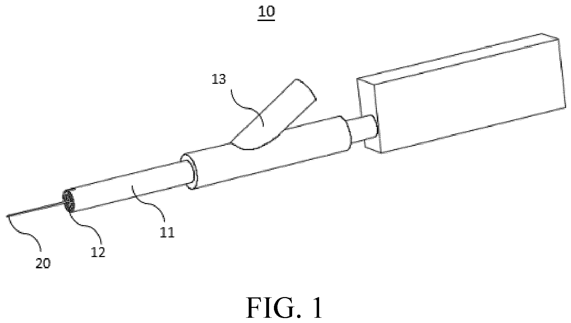

Referring to FIG. 1, this application provides a thrombectomy device 10 for excising a thrombus in a blood vessel. The thrombectomy device 10 is guided by a guide wire 20 into a designated position of a blood vessel to excise a thrombus. The types of the thrombus include but are not limited to a cerebral thrombus, a pulmonary thrombus, and a cardiac thrombus.

The thrombectomy device 10 includes a catheter 11, an excision assembly 12, and a suction assembly 13. The catheter 11 extends into the blood vessel. One end of the catheter moves to a location of the thrombus. The excision assembly 12 is at least partially driven by the catheter 11 to move to the location of the thrombus to excise the thrombus. The suction assembly 13 is communicated with an inner cavity of the catheter 11 and sucks air from the catheter 11 to make the interior of the catheter 11 in a negative pressure state when the thrombectomy device 10 is operated. Under the action of the negative pressure, when the thrombus is sucked into the catheter 11 at an end surface of the catheter 11 extending into the blood vessel, the excision assembly 12 excises the thrombus. The excised thrombus is sucked away under the action of the suction force generated by the suction assembly 13.

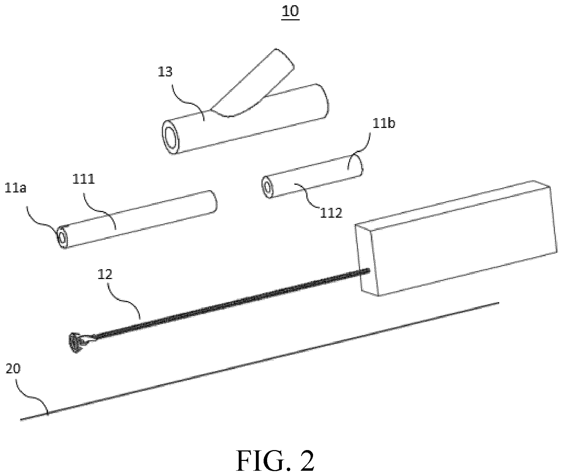

Referring to FIG. 2, the catheter 11 is a circular hollow tube made of a flexible material. The catheter 11 may extend into the blood vessel and deform in accordance with the direction of the blood vessel to facilitate delivery in the curved blood vessel. The catheter 11 includes a first catheter 111 and a second catheter 112. The first catheter 111 and the second catheter 112 are communicated by the suction assembly 13. The catheter 11 has opposite ends in an axial direction. For convenience of description, the end of the catheter 11 extending into the blood vessel is defined as a distal end 11a, and the other end is defined as a proximal end 11b. It will be understood that the catheter 11 may also not be composed of the segmented first catheter 111 and second catheter 112, but rather a complete catheter. An opening (not shown) is provided on a side wall of the catheter. The suction assembly 13 is communicated with the catheter 11 at the opening.

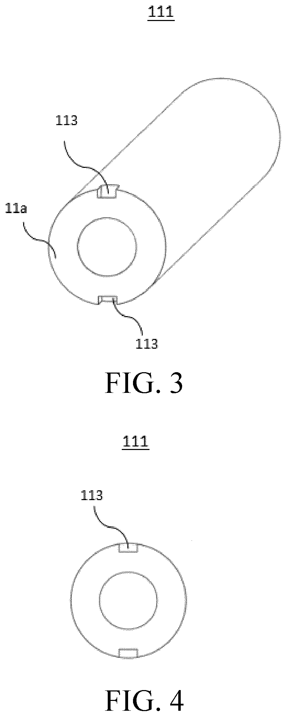

Referring to FIG. 3 to FIG. 5, clamping grooves 113 are provided on an outer side wall of the distal end 11a of the catheter 11, namely the end of the first catheter 111 extending into the blood vessel, and are configured to clamp a portion of components of the excision assembly 12. The clamping grooves 113 are wedge-shaped. There are at least two clamping grooves 113 provided centrosymmetrically about the axis of the catheter 11. It will be understood that the shape of the clamping grooves 113 is not limited and the clamping grooves may be triangular prism-shaped, trapezoidal, or of other regular or irregular shapes as long as the clamping function can be realized. As a specific embodiment of the clamping grooves 113 for clamping, the depth of the clamping grooves 113 is gradually increased from the distal end 11a to the proximal end 1 1b in the axial direction of the catheter 11 to enable clamping of a portion of the components of the excision assembly 12.

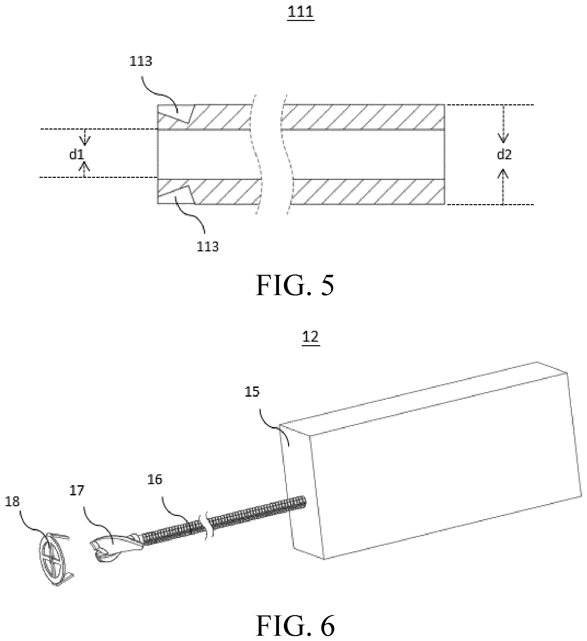

Referring to FIG. 5, as an embodiment, the length of the catheter 11 is 1.5 mm to 2.5 m, an inner diameter d1 of the catheter 11 is 1.3 mm to 1.9 mm, and an outer diameter d2 (diameter) is 2.8 mm to 3.4 mm. Preferably, the outer diameter of the catheter 11 is 3.1 mm to 3.3 mm, optionally 3.18 mm. The inner diameter of the catheter 11 is 1.5 mm to 1.7 mm, optionally 1.59 mm.

Referring to FIG. 6, the excision assembly 12 includes a power source 15, a transmission shaft 16, a cutter head 17, and a cutter net 18. One end of the transmission shaft 16 is connected to the power source 15, and the other end is connected to the cutter head 17. The power source 15 drives the cutter head 17 to rotate through the transmission shaft 16. The cutter net 18 is fixed to an end surface of the distal end 11a of the catheter 11 (namely, the front end surface of the catheter 11). During the operation of the thrombectomy device 10, a front end of the cutter head 17 and the cutter net 18 cooperate to form at least one cutting structure. The cutting structure generates a shear force applied to the thrombus, thereby excising the thrombus. The cutting structure includes a first cutter edge and a second cutter edge (described in more detail later) cooperating with each other. The first cutter edge is located on the cutter head 17, and the second cutter edge is located on the cutter net 18.

The power source 15 may be any device such as a drive motor capable of driving a member connected thereto to rotate. The power source 15 includes a power output shaft and a first sealing member (both not shown). The power output shaft of the power source 15 is connected to one end of the transmission shaft 16. The first sealing member is arranged between the transmission shaft 16 and the catheter 11 for sealing the proximal end 11b of the catheter 11 so that the interior of the catheter 11 is a relatively closed space.

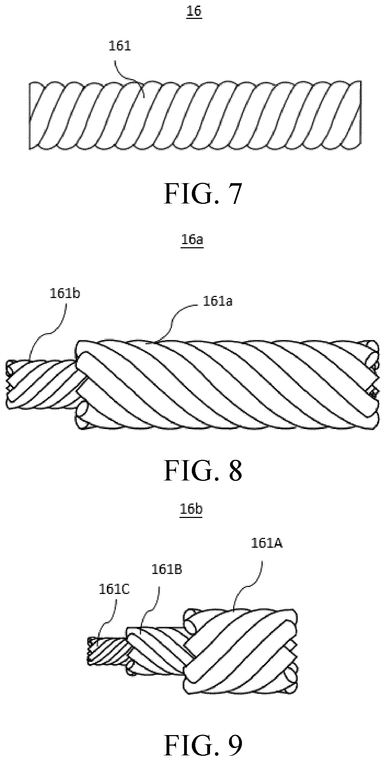

Referring to FIG. 7, the transmission shaft 16 is flexible to facilitate delivery in the curved blood vessel. The transmission shaft 16 has an internal hollow structure. That is, a first through hole is formed. The hollow structure penetrates through opposite ends of the transmission shaft 16. The hollow structure allows passage of the guide wire 20.

As an embodiment, the transmission shaft 16 includes m spiral wire tubes stacked in sequence from the inside to the outside. Each spiral wire tube is formed by winding n filaments 161 side by side, and the hollow structure is formed along the axis of the transmission shaft after winding. When there are a plurality of spiral wire tubes, the winding directions of the adjacent two spiral wire tubes are opposite. m is a positive integer greater than or equal to 1, and n is a positive integer greater than or equal to 2. Preferably, the value of m is not greater than 6, and the value of n is not greater than 18. Further preferably, m is greater than or equal to 1 and is less than or equal to 3, and n is greater than or equal to 2 and is less than or equal to 9. Under this parameter, the advantages of better bearing capacity, less fracture, and better safety performance of the transmission shaft 16 are more prominent.

As shown in FIG. 7, the transmission shaft 16 is formed by winding a single layer of filaments 161 side by side. As shown in FIG. 8, the transmission shaft 16 is formed by winding two layers of filaments side by side, and specifically includes outer filaments 161a and inner filaments 161b which are stacked and wound in opposite directions. As shown in FIG. 9, the transmission shaft 16 is formed by winding three layers of filaments side by side, and specifically includes outer filaments 161A, middle filaments 161B, and inner filaments 161B stacked. The adjacent filaments are wound in opposite directions.

As a preferred embodiment, the filament wires 161 are metal wires, which are 316LVM stainless steel wires or 40CrMoV alloy steel wires or 35NLT alloy wires or 304V alloy steel wires.

As a preferred embodiment, the transmission shaft 16 has an outer diameter of 0.55 mm to 0.75 mm and an inner diameter (namely, the diameter of the hollow structure) of 0.01 mm to 0.28 mm. Further preferably, the transmission shaft 16 has an outer diameter of 0.6 mm to 0.7 mm and an inner diameter of 0.15 mm to 0.23 mm. As a specific embodiment, the transmission shaft 16 has an inner diameter of 0.29 mm and an outer diameter of 0.65 mm. m is equal to 1, and n is equal to 3. That is, the transmission shaft 16 is formed by winding three metal wires with a diameter of 0.18 mm. Under the aforementioned parameters, the transmission shaft 16 is not easy to break, and the torque transmissibility and the bearing capacity are better. A distance between the transmission shaft 16 and the inner wall of the catheter 11 is provided to determine the maximum size of the excised thrombus which can be passed. In this application, the distance is 0.3 mm to 0.7 mm.

The transmission shaft 16, which is formed by winding n filaments 161 side by side and in which two adjacent spiral wire tubes are wound in opposite directions, has excellent torque transmission performance and bearing capacity on the one hand, and is not prone to fracture on the other hand, and has excellent safety performance.

A gap of a suitable size is provided between the transmission shaft 16 and the catheter 11 so that the excised thrombus can enter the suction assembly 13 through the gap. As an embodiment, the size of the gap between the transmission shaft 16 and the catheter 11 is 0.3 mm to 0.7 mm.

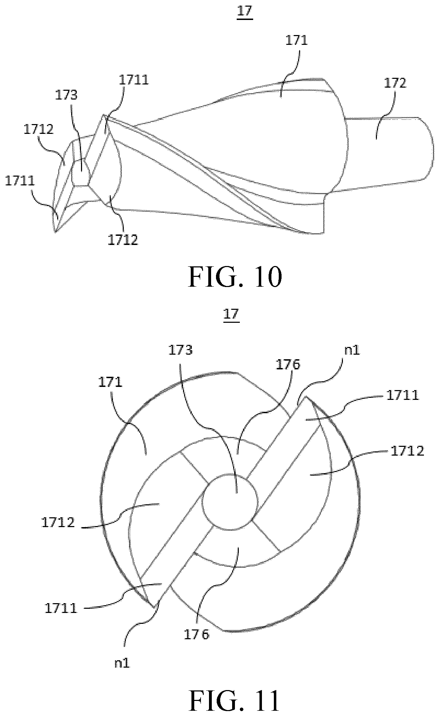

Referring to FIG. 10 to FIG. 12, the cutter head 17 may be any structure of the cutter head 17 so long as the cutter head can be meshed with the cutter net 18 to form the cutting structure, which belongs to the application concept protected by this application.

The cutter head 17 is made of metal. The cutter head 17 is provided with a second through hole 173 allowing passage of the guide wire 20 in the axial direction, and the second through hole 173 penetrates through the cutter head 17. The cutter head 17 includes an integrally formed connecting portion 172 and at least one cutter edge body 171. Each cutter edge body 171 cooperates with the cutter net 18 to form the cutting structure. When there are a plurality of cutter edge bodies 171, the cutter edge bodies 171 are arranged symmetrically about the second through hole 173. In this application, the cutter head 17 preferably includes at least two cutter edge bodies 171 to improve the efficiency of thrombectomy and the stability of the operation of the cutter head 17. In the figures, two cutter edge bodies 171 are included. The two cutter edge bodies 171 are arranged centrosymmetrically about the second through hole 173 (namely, the axis of rotation of the cutter head 17). The top of the cutter edge body 171 close to the cutter net 18 includes a first surface 1711 and a second surface 1712. The first surface 1711 and a second surface 1712 are arranged in the rotation direction of the cutter head 17 in sequence and are planar. That is, the second surface 1712 is arranged on the rear side of the first surface 1711, and the second surface is inclined away from the cutter net 18 in a direction opposite to the rotation direction of the cutter head 17. The plane on which the cutter head 17 rotates (namely, a plane perpendicular to the rotation axis of the cutter head 17) is defined as a reference plane A. The first surface 1711 and the second surface 1712 are inclined to the reference plane A. Included angles between the first surface 1711 and the second surface 1712 and the reference plane A are acute angles a1 and a2, respectively. As an embodiment, a1 is less than a2, and the surface of the cutter edge body 171 closest to the cutter net 18 is the first surface 1711. As a further embodiment, a1 is greater than 0° and is less than or equal to 5°, and a2 is greater than 5° and is less than or equal to 15°. An edge n1 of the first surface 1711 away from the second surface 1712 is the first cutter edge of the cutting structure. The first cutter edge and the cutter net 18 cooperate to excise the thrombus. In a radial direction of the cutter head 17, a gap between the cutter head 17 and the inner wall of the catheter 11 is provided to prevent the cutter head 17 from colliding with the catheter 11 and causing the catheter 11 to be damaged. The gap is greater than or equal to 0.03 mm. Further preferably, the gap is 0.05 mm.

Referring further to FIG. 11, a space 176 is provided between the adjacent cutter edge bodies 171. Specifically, the space is located between the first surface 1711 of the cutter edge body 171 and the second surface 1712 of another cutter edge body 171 adjacent thereto, that is, on the side of the first surface 1711 away from the second surface 1712. The space 176 is provided to facilitate the removal of the thrombus while also facilitating the sharpening of the edge n1. Preferably, the cutter edge body 171 is integrally formed in a spiral shape. A spiral protrusion 174 and a spiral groove 175 arranged at intervals are formed on an outer surface of the cutter head 17. The spiral protrusion 174 and the spiral groove 175 spirally extend in an extending direction of the catheter 11. The end of the spiral groove 175 forms a space 176, or the spiral groove 175 may be considered as a space 176. The cutter edge body 171 and the space 176 for removing the excised thrombus extend spirally in the axial direction of the catheter 11. The cutter edge body 171 extends spirally (multi-head spiral structure), which is beneficial to processing. More importantly, the propulsion force of the cutter head 17 can be effectively improved to increase the thrombectomy effect.

When the cutter head 17 approaches the cutter net 18, the edge n1 (first cutter edge) of the first surface 1711 of the cutter edge body 171 first contacts the cutter net 18. The first cutter edge cooperates with the cutter net 18 to generate shear force on the thrombus sucked into the catheter 11 from the cutter net 18, so as to excise the thrombus. It will be understood that the first cutter edge is not limited to the edge of the first surface 1711 away from the second surface 1712 but may also be a common edge of the first surface 1711 and the second surface 1712. At this moment, a1 is greater than a2. As a modification, the first surface 1711 may be parallel to the reference plane A. That is, a1 is equal to 0. As yet another modification, the second surface 1712 may be omitted. That is, a top surface of the cutter head 17 close to the cutter net 18 includes only the first surface 1711. It will also be understood that the first surface 1711 is coplanar with the second surface 1712 in this case.

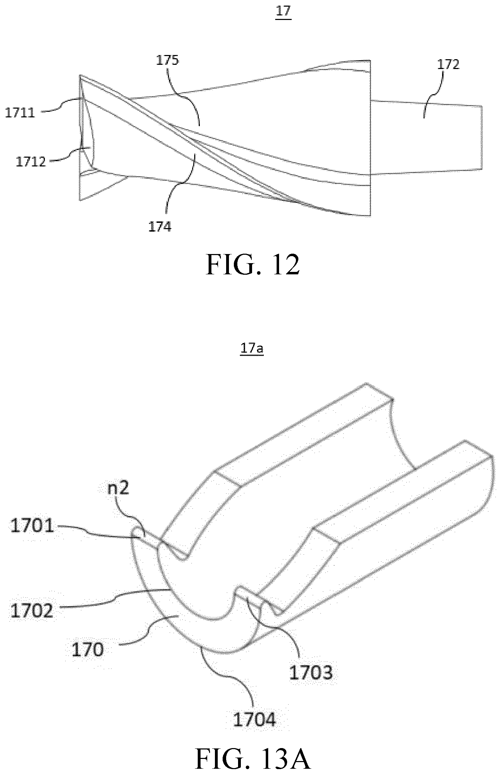

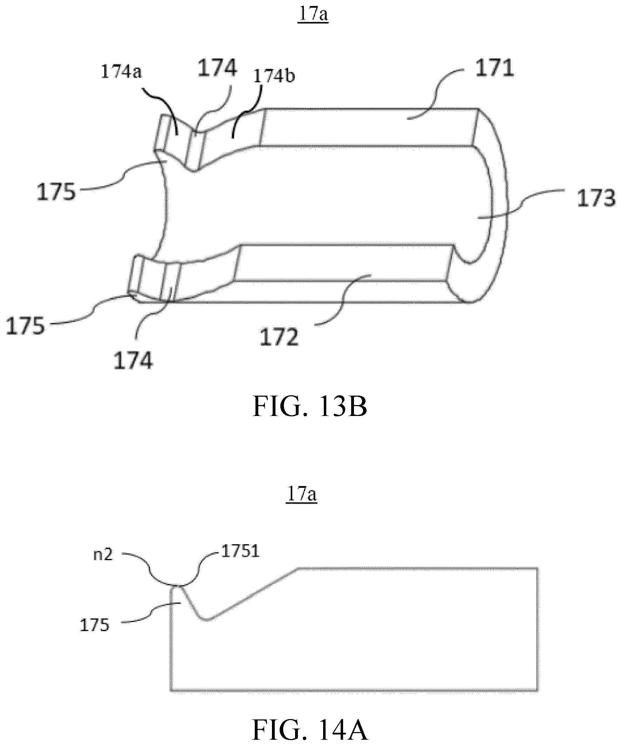

Referring to FIG. 13A to FIG. 14B, as another embodiment of the cutter head 17, another form of the cutter head 17a is shown. An edge n2 of the other form of the cutter head 17a is the first cutter edge.

The cutter head 17a includes a cutter edge body. A surface of the cutter edge body close to the cutter net 18 is the first surface 170. The first surface 170 is a plane. The first surface 170 has a first edge 1701, a second edge 1702, a third edge 1703, and a fourth edge 1704, which are connected in sequence. The second edge 1702 and the fourth edge 1704 are arc-shaped. The first edge 1701 and the third edge 1703 are straight. The first edge 1701 and the third edge 1703 are connected to both ends of the second edge 1702 and the fourth edge 1704 respectively. The first surface 170 is parallel to the reference plane A, or an included angle is provided between the first surface 170 and the reference plane A (at this moment, the first surface 170 may be provided as a concave surface, or may be formed by a plurality of different surfaces) and the first edge 1701 and the third edge 1703 are parts of the cutter head 17 closest to the cutter net 18. Preferably, a projection of the first plane 170 on the reference plane A is semicircular.

The cutter edge body includes a first plane 171 and a second plane 172 extending in the axial direction of the cutter head 17a, and a cambered surface 173 connecting the first plane 171 and the second plane 172. A notch 174 is provided at the end of the first plane 171 and/or the second plane 172 close to the cutter net 18. A tip structure 175 is formed on the side of the notch 174 close to the cutter net 18. The tip structure 175 includes a tip 1751. The tip 1751 is the aforementioned first edge 1701 and third edge 1703 extending radially. The cambered surface 173 may also be an inclined surface or another irregular surface. Preferably, the first plane 171 and the second plane 172 are also parallel to the radial direction of the cutter head 17.

The notch has a first notch surface 174a and a second notch surface 174b. The first notch surface 174a and the second notch surface 174b are smoothly connected by a circular arc. An included angle between the first notch surface 174a and the second notch surface 174b is 80°-120°, preferably 90°-100°. The first notch surface 174a is a side surface of the tip structure 175. An included angle between the first notch surface 174a and the reference plane A is 15°-35°, preferably 20°-30°. Under the parameters, the sharpness of the cutter head 17a and the removal smoothness of the excised thrombus can be taken into account.

The cutter head 17a and the cutter net 18 form a cutting structure for shearing the thrombus. The cutting structure includes a first cutter edge and a second cutter edge. The first edge 1701 and the third edge 1703 form the first cutter edge. That is, the tip 1751 forms the first cutter edge. The second cutter edge is located on the cutter net 18. When the cutter head 17a approaches the cutter net 18, the first cutter edge of the cutter head 17a first contacts the cutter net 18. The first cutter edge cooperates with the cutter net 18 to generate shear force on the thrombus sucked into the catheter from the cutter net 18, so as to excise the thrombus.

The first plane 171 is coplanar with the second plane 712. Extension lines of the first cutter edges on the two tip structures 175 are collinear and pass through the center of rotation of the cutter head 17. The first plane 171 and the second plane 172 are located in a plane higher than the line of the first cutter edge, and a height difference between the plane of the first plane 171 and the second plane 172 and the line of the first cutter edge is 10% to 30% of the outer diameter of the cutter head 17. This design is convenient for processing and manufacturing, and is beneficial to reliable fixation with the transmission shaft 16. The length of the first cutter edge is 20% to 40% of the outer diameter of the cutter head 17, thus providing an appropriate amount of excision and reducing the likelihood of contact between the first cutter edge and the catheter 11.

As an embodiment, the cutter head 17 is made of metal. As a modified embodiment, the notch 174 may be omitted.

The transmission shaft 16 is fitted on the connecting portion 172 and fixedly connected to the connecting portion. The connection may be achieved by welding, clamping, bonding, or any other way in which the transmission shaft 16 and the cutter head 17 can be fixedly connected. As a modification, a countersink (not shown) coaxial with the second through hole 173 is provided at the end of the cutter head 17 away from the cutter net 18. The radial dimension of the countersink is greater than the radial dimension of the second through hole 173. The countersink is configured to mount the transmission shaft 16. The transmission shaft 16 is fixed in the countersink in any way such as welding, clamping or bonding, which can fixedly connect the transmission shaft 16 with the cutter head 17. The axis of the transmission shaft 16 is coaxial with the second through hole 173. It will be understood that the countersink may be omitted and the transmission shaft 16 is directly fixed to the side wall of the second through hole 173.

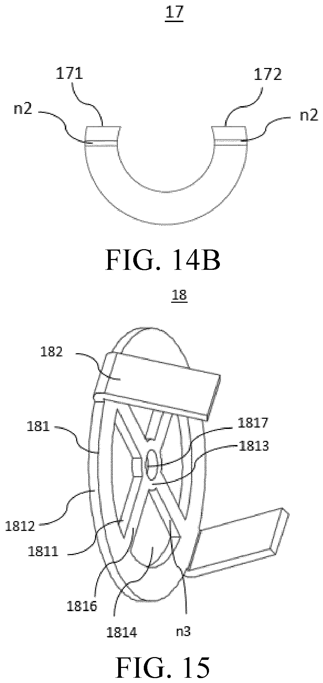

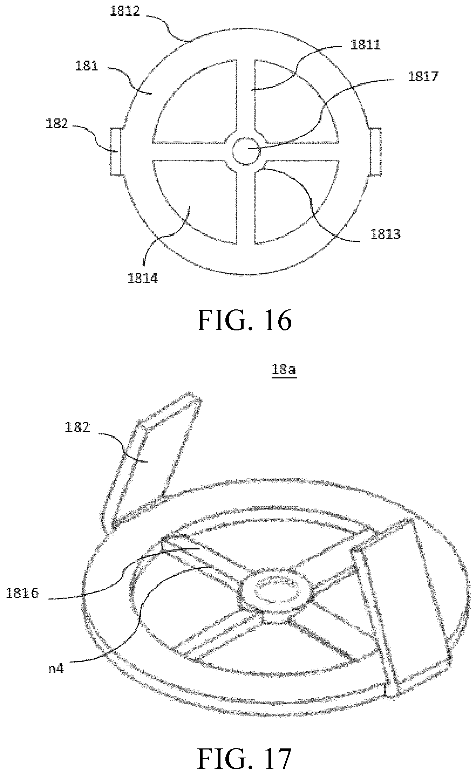

Referring to FIG. 15 and FIG. 16, the cutter net 18 includes a cutter net body 181 and a fixing member 182. The cutter net body 181 and the fixing member 182 are fixedly connected. The fixing connection may be formed by integral molding, or may be formed by welding after split molding. The cutter net 18 is fixed to the catheter 11 by the fixing member 182. Specifically, the fixing member 182 is clamped in the clamping groove 113 of the catheter 11. There are at least two fixing members 182 arranged symmetrically about the center of the cutter net 18 and are in positional correspondence with the clamping grooves 113 on the catheter 11. The number of fixing members 182 is two in this application. The fixing member 182 is specifically a horizontally-shaped sheet body. One end of the fixing member is connected to the cutter net body 181, and the other end is inclined toward the center of the cutter net 18. The distance from the fixing member 182 to the axis of the cutter net 18 is gradually reduced from the connecting end of the fixing member 182 with the cutter net body 181 to the end far away from the cutter net body 181. When the fixing member 182 is clamped in the clamping groove 113, the fixing member 182 is clamped on the inner wall of the clamping groove 113. The fixing member 182 and the clamping groove 113 are matched in shape and size to achieve the beneficial effects of simple clamping structure, simple clamping operation, stable connection, and convenient manufacture.

It will be understood that the fixing member 182 is not limited to being connected to the catheter 11 by clamping, but may also be connected to the catheter 11 in other possible ways such as bonding. The clamping mode is optimal.

The cutter net body 181 includes an outer annular structure 1812, an inner annular structure 1813, and connecting ribs 1811. The connecting ribs 1811 are integrally formed with the outer annular structure 1812 and the inner annular structure 1813. The outer annular structure 1812 and the inner annular structure 1813 are circular and coaxially arranged, and a third through hole 1817 allowing passage of the guide wire 20 is provided in the middle of the inner annular structure 1813. One end of each connecting rib 1811 is fixed to the outer annular structure 1812, and the other end is fixed to the inner annular structure 1813. The connecting ribs 1811 extend in the radial direction of the inner annular structure 1813 or the outer annular structure 1812, and are uniformly distributed between the outer annular structure 1812 and the inner annular structure 1813. The number of the connecting ribs 1811 is not limited, but at least 2. The number of the connecting ribs 1811 is 4 in this application. A hollowed-out portion 1814 is formed between the adjacent connecting ribs 1811. The hollowed-out portion 1814 axially penetrates through the cutter net 18 for the thrombus to enter the catheter 11. The fixing member 182 is connected to the outer side surface of the outer annular structure 1812. The outer diameter of the outer annular structure 1812 is slightly smaller than the outer diameter of the catheter 11 (the difference between the two diameters is 0.01 to 0.8 mm). When the cutter net 18 is fixed to the end surface of the distal end 11a of the catheter 11, the outer annular structure 1812 and the inner annular structure 1813 coincide with the axis of the catheter 11. The inner annular structure 1813 is located at the center of the outer annular structure 1812 or the catheter 11. It may be considered that one end of the connecting ribs 1811 is fixedly connected together at the center of the outer annular structure 1812 or the catheter 11. Preferably, the maximum size of the cutter net 18 in the radial direction is less than or equal to the outer diameter of the catheter 11. In this way, when the catheter 11 drives the cutter net 18 to move in the blood vessel, the cutter net 18 does not easily contact the inner wall of the blood vessel, thus effectively protecting the blood vessel and preventing the cutter net 18 from easily falling off due to resistance.

Referring to FIG. 15 and FIG. 16, the connecting ribs 1811 are strips of equal thickness. The surface of each connecting rib 1811 close to the cutter head 17 is parallel to the reference plane A. An edge n3 of the connecting rib 1811 close to the cutter head 17 in the rotation direction of the cutter head 17 and first contacting or approaching the first cutter edge during the rotation of the cutter head 17 is the second cutter edge. That is, a part of the edge defining the hollowed-out portion 1841 close to the cutter head 17 forms the second cutter edge. When the thrombectomy device is operated, the cutter head 17 rotates and the connecting ribs 1811 are fixed. That is, the first cutter edge rotates and the second cutter edge is fixed. The first cutter edge and the second cutter edge cooperate to excise the thrombus. Different connecting ribs 1811 may cooperate with the cutter head 17 to form the cutting structure. The first cutter edge and the second cutter edge are horizontally-shaped and extend in the radial direction of the catheter 11.

As a preferred embodiment, the cutter net body 181 has the same thickness. That is, the outer annular structure 1812, the inner annular structure 1813, and the connecting ribs 1811 have the same thickness. As another embodiment, the cutter net 18 has the same thickness. That is, the outer annular structure 1812, the inner annular structure 1813, the connecting ribs 1811, and the fixing member 182 have the same thickness, and may be manufactured by the same plate. The cutter net body 181 and a corresponding part of the fixing member are processed by cutting the plate, and then the corresponding part of the fixing member is bent to form the fixing member 182, which has the advantages of simplified manufacturing process and good overall strength of products.

Referring to FIG. 17, as an embodiment, the connecting ribs 1811 are strips of unequal thickness. The surface of the connecting rib 1811 close to the cutter head 17 is inclined to the reference plane A. An edge n4 of the connecting rib 1811 closest to the cutter head 17 is the second cutter edge. That is, a partial edge defining the hollowed-out portion forms the second cutter edge. The second cutter edge may be sharpened to improve the efficiency of thrombectomy. Preferably, the inclined plane is parallel to the first surface 1711.

The cutter net 18 is preferably made of metal and is manufactured by punching and bending processes. That is, the cutter net 18 is punched by a metal sheet to obtain the cutter net body 181 and a pre-fixing member connected to the cutter net body 181. The pre-fixing member is bent to form the fixing member 182. As a modification, the outer annular structure 1812 of the cutter net body 181 may be omitted, and the connecting ribs 1811 are directly connected to the fixing member 182.

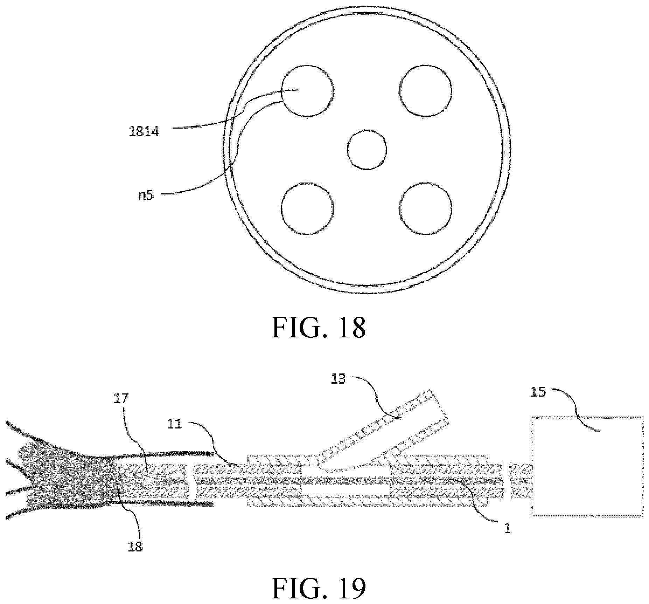

It will be understood that the structure of the cutter net body 181 is not limited to the above embodiment. As shown in FIG. 18, the cutter net body 181 is a plate-like body having a hollowed-out portion 1814. The hollowed-out portion 1814 axially penetrates through the cutter net body 181. A partial edge n5 on the cutter net 18 that defines the hollowed-out portion 1814 (and close to the cutter head 17) is the second cutter edge. In this case, a portion outside the hollowed-out portion 1814 is equivalent to the outer annular structure.

In a non-operation state, a certain distance between the cutter head 17 and the cutter net 18 is provided to facilitate the entry of the thrombus into the catheter 11 from the hollowed-out portion 1814. The distance is less than or equal to 2 mm, and further preferably less than or equal to 1 mm. In an operation state, the power source 15 may drive the cutter head 17 toward the cutter net 18 by driving the transmission shaft 16. The cutter net 18 contacts the cutter head 17 to generate a stronger shear force. It will be understood that in the operation state of the thrombectomy device, a small gap is also allowed between the cutter net 18 and the cutter head 17.

The suction assembly 13 is configured to suck air from the catheter 11 to make the interior of the catheter 11 in a negative pressure state. The suction assembly 13 includes a three-way pipe, a suction port, a thrombus collection chamber, and a second sealing member (not labeled). The suction assembly 13 is connected to the suction port, the first catheter 111, and the second catheter 112 through the three-way pipe. The second sealing member is arranged at the junction of the three-way pipe, the suction port, and the catheter 11 to ensure the sealing connection. The excised thrombus is sucked through the suction port under the negative pressure and then enters the thrombus collection chamber. As an embodiment, the suction assembly 13 includes a vacuum pump.

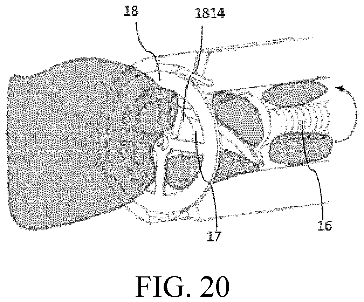

Referring to FIG. 19 and FIG. 20, when the thrombectomy device 10 is operated, the thrombectomy device 10 is penetrated on the guide wire 20. that is, the guide wire 20 passes through the first through hole, the second through hole 173, and the third through hole 1817 of the transmission shaft 16. The thrombectomy device 10 enters the body along the guide wire 20 and reaches the thrombus site. Under the action of external force, the transmission shaft 16 and the cutter head 17 move in the axial direction inside the catheter 11, and then the cutter head 17 is delivered to the vicinity of the cutter net 18. The guide wire 20 guides the thrombectomy device 10 to move to the thrombus site. Before the thrombus is excised, the cutter head 17 may be stationary or rotate at a low speed, preferably at a speed of rotation of 0 to 20,000 rpm. When the thrombus is to be excised, the power source 15 drives the cutter head 17 to rotate at a high speed with the guide wire 20 as the axis through the transmission shaft 16, and at the same time makes the cutter head 17 approach the cutter net 18 so that the cutter head and the cutter net are in contact or there is a slight gap therebetween. The first cutter edge on the cutter head 17 and the second cutter edge on the cutter net 18 cooperate to generate shear force on the thrombus coming in from the hollowed-out portion 1814, thereby completing excision of the thrombus. During the excision process, the cutter head 17 rotates at a high speed, preferably at a speed of rotation of 60,000 rpm to 200,000 rpm.

It will be understood by those skilled in the art that the above preferred schemes can be freely combined and superimposed without conflict.

It should be understood that the above implementations are exemplary only and not limiting and that various obvious or equivalent modifications or substitutions made with respect to the above details by those skilled in the art will be included within the scope of the claims of this application without departing from the basic principles of this application.

## Claims

1. A thrombectomy device for excising a thrombus in a blood vessel, the thrombectomy device comprising a catheter and an excision assembly, wherein the excision assembly comprises a cutter net and a cutter head; the cutter net is fixed to a front end surface of the catheter, and the cutter head is located inside the catheter; a hollowed-out portion is arranged on the cutter net in an axially-penetrating manner; the cutter head is drivable to rotate in the catheter; a front end of the cutter head cooperates with the cutter net to form a cutting structure to excise a thrombus sucked from the hollowed-out portion;
the cutting structure comprises a first cutter edge and a second cutter edge; the first cutter edge is located on the cutter head, and the second cutter edge is located on the cutter net; the cutter head comprises at least one cutter edge body, a front end of the cutter edge body closest to the cutter net is a first surface, and an edge of the first surface forms the first cutter edge; a partial edge of the hollowed-out portion close to the cutter head forms the second cutter edge; and when the thrombectomy device is operated, the first cutter edge rotates, the second cutter edge is fixed, and the first cutter edge and the second cutter edge cooperate to excise the thrombus.

2. The thrombectomy device according to claim 1, wherein the cutter edge body comprises a second surface adjacent to the first surface, the second surface is arranged on the rear side of the first surface in a rotation direction of the cutter head, and the second surface is inclined away from the cutter net in a direction opposite to the rotation direction of the cutter head.

3. The thrombectomy device according to claim 1, wherein the cutter head comprises at least two cutter edge bodies, the at least two cutter edge bodies are distributed centrosymmetrically about a rotation axis of the cutter head, and a space for removing the excised thrombus is provided between the adjacent two cutter edge bodies in the rotation direction of the cutter head.

4. The thrombectomy device according to claim 3, wherein the cutter edge body and the space for removing the excised thrombus extend spirally in an axial direction of the catheter.

5. The thrombectomy device according to claim 1, wherein the cutter head is a semicircular ring-like body; the first surface has a first edge, a second edge, a third edge, and a fourth edge, which are connected in sequence; the second edge and the fourth edge are arc-shaped, and the first edge and the third edge are connected to both ends of the second edge and the fourth edge respectively; and the first edge and the third edge form the first cutter edge.

6. The thrombectomy device according to claim 5, wherein the cutter head has a first plane and a second plane extending in an axial direction of the cutter head, and a cambered surface connecting the first plane and the second plane; and the ends of the first plane and the second plane close to the cutter net are provided with a notch separately, a tip structure is formed on the side of the notch close to the cutter net, and the tip structure comprises a tip that forms the first cutter edge.

7. The thrombectomy device according to claim 1, wherein the first surface is parallel to a reference plane A or an included angle is formed between the first surface and the reference plane A; and the reference plane A is perpendicular to a rotation axis of the cutter head, the first surface is a plane, and the first cutter edge is a part of the cutter head closest to the cutter net.

8. The thrombectomy device according to claim 1, wherein the cutter net comprises a cutter net body and a fixing member; the cutter net body is fixedly connected to the fixing member, and the cutter net is fixed to the catheter through the fixing member; the cutter net body comprises an outer annular structure and at least two connecting ribs; the connecting ribs extend in a radial direction of the catheter, and radial outer ends of the connecting ribs are connected to the outer annular structure; the hollowed-out portion is formed between the adjacent connecting ribs; the connecting ribs and the cutter head cooperate to form the cutting structure; and partial edges of the connecting ribs close to the cutter head form the second cutter edge.

9. The thrombectomy device according to claim 8, wherein the cutter net comprises at least two fixing members; one end of each fixing member is fixedly connected to the outer annular structure, the other end of the fixing member is a free end, the distance from the fixing member to the axis of the cutter net is gradually reduced from the connecting end of the fixing member with the cutter net body to the free end; clamping grooves quantitatively matched with the fixing members are provided on an outer wall of the catheter, and the depth of the clamping grooves is gradually increased from the end close to the cutter net to the end far away from the cutter net in the axial direction of the catheter; and when the fixing members are fixed in the clamping grooves, the fixing members are clamped on the catheter.

10. The thrombectomy device according to claim 1, wherein the cutter head and the cutter net are provided with a central through hole in the axial direction of the catheter for allowing passage of a guide wire.

11. The thrombectomy device according to claim 1, further comprising a suction assembly, wherein the suction assembly is communicated with an inner cavity of the catheter and sucks air from the catheter to make the interior of the catheter in a negative pressure state when the thrombectomy device is operated, and the excision assembly further comprises a power source that drives the cutter head to rotate in the catheter.

12. The thrombectomy device according to claim 11, wherein the excision assembly comprises a transmission shaft; the power source and the cutter head are connected through the transmission shaft; before the thrombus is excised, a certain distance is provided between the cutter head and the cutter net, and the distance is less than or equal to 1 mm; and when the thrombus is excised, the cutter head approaches the cutter net and forms the cutting structure for excising the thrombus.

13. The thrombectomy device according to claim 1, wherein the speed of rotation of the cutter head is 60,000 rpm to 200,000 rpm when the cutter head excises the thrombus.
